# EUROPEAN PATENT SPECIFICATION

(11) **EP 1 496 874 B1**
(45) Date of publication and mention of the grant of the patent: **01.08.2007**
(21) Application number: 03712555.6
(22) Date of filing: 15.04.2003
(51) Int. Cl.: A61K 9/70, A61K 31/216

(54) **TRANSDERMAL PATCHES HAVING A SILICONIC ADHESIVE MATRIX STABILIZED WITH METHACRYLIC COPOLYMERS**
TRANSDERMALE PFLASTER MIT EINER ADHÄSIVEN SILICONMATRIX STABILISIERT MIT METHACRYL COPOLYMEREN
TIMBRES TRANSDERMIQUES COMPRENANT UNE MATRICE SILICONE ADHESIVE STABILISEE AVEC DES POLYMERES METHACRYLIQUES

(30) Priority: 15.04.2002 IT MI20020798
(43) Date of publication of application: 19.01.2005
(73) Proprietor: F.T. Holding S.A., 1150 Luxembourg (LU)
(72) Inventor: CILURZO, Francesco, I-20154 Milano (IT); TOSI, Leila, I-20159 Milano (IT)
(74) Representative: Gervasi, Gemma
(86) International application number: PCT/IB2003/001459
(87) International publication number: WO 2003/086370

(56) References cited:
- WO-A-02/45699
- WO-A-99/48493
- FR-A- 2 749 586
- US-B1- 6 335 031
- KOTIYAN PRAMILA N ET AL: "Eudragits: Role as crystallization inhibitors in drug-in-adhesive transdermal systems of estradiol." EUROPEAN JOURNAL OF PHARMACEUTICS AND BIOPHARMACEUTICS, vol. 52, no. 2, September 2001 (2001-09), pages 173-180, XP002249753 ISSN: 0939-6411

## Description

### FIELD OF THE INVENTION

The present invention relates to patches suitable for transdermal or topical administration containing adhesive matrices based on storage-stable silicone polymers.

### STATE OF THE ART

The therapeutic treatment of patients with pharmaceutically acceptable substances is commonly effected by periodic administration of defined drug doses during the 24 hours of the day. However the classical administration routes such as the oral or injective ones require repeated administration of high dosages to ensure an effective drug level in the body. Controlled drug release by intravenous injection compared with oral administration avoids discontinuous administration and hence the relative release, by maintaining a constant and prolonged drug level, while at the same time avoiding the first stage of hepatic metabolism.

With this in mind, transdermal administration was conceived and developed for systemic medication and local therapy, and presents undoubted advantages not only compared with said traditional systemic applications, but also compared with more conventional topical formulations such as ointments, unguents and creams.

In this respect, this latter provides the advantages of controlled direct entry of a drug into the blood circulation associated with indisputable ease of application by the patient. Whether administering a drug by systemic medication or by local treatment, transdermal administration uses the intact skin as the entry portal and the application site for the drug.

Various types of transdermal systems have been conceived and developed.

The first is the reservoir device. In this system the support layer and a membrane able to control the drug release form a drug deposit or reservoir layer which contains the active principle in liquid or gel form. The permeable membrane controls the rate at which the drug is released. As the drug solution is saturated, the release through the membrane is constant with time. An inert permeable film covers either the entire surface in contact with the skin or only a part of the patch depending on the compatibility of the adhesive with the vehicle and the drug formulation.

The second type of device incorporates a polymer matrix into which the active principle is loaded and within which it has to diffuse through the polymer network from a homogeneous continuous phase or a dispersed phase of the drug in the polymer or as drug microreservoirs uniformly dispersed in the polymer phase. The diffusion rate controls the drug release rate which decreases with time while draining the matrix a part thereof by the part in contact with the skin. As in the case of the reservoir device, adhesion to the skin is provided by an adhesive layer which covers said polymer or alternatively by an adhesive edge.

The third one is the simplest type, in which the adhesive matrix contains the drug and is applied directly onto the skin. Pressure sensitive adhesives (PSA) play a key role in all three said types of device by ensuring intimate and reliable contact between the transdermal patch and the surface of the skin. In this respect, the patch is flexible and adheres to the skin by applying a slight pressure, without causing irritation, for a time period between 1 and 7 days, without leaving residues once removed. In addition the PSA must be permeable both to the active principles and to the relative absorption enhancers.

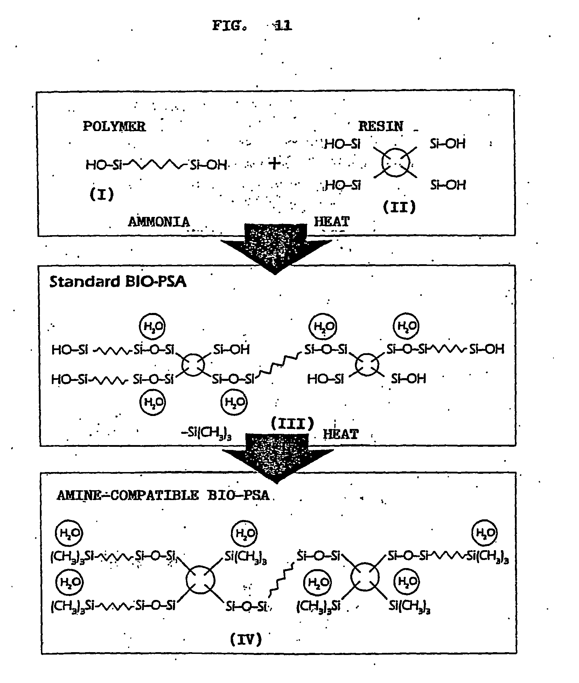

The silicone PSAs (lll) described in Figure 11 are the condensation products of the polymer (I) shown in Figure 11 which presents silanols as terminal groups, with the silicate resin (II). (I) is a polydimethylsiloxane of low viscosity (12,000-15,000 Cps) or a rubber with silanol functionality in the main chain. The resin (II) is a soluble network of silicates. The ratio resin(II)/polymer(I) in the polycondensate (III) determines the optimum balance between adhesive and cohesive properties. In this respect, on increasing the content of polymer (I), (III) presents higher viscosity and lower resistance to shear, whereas in contrast a high content of resin (II) in (III) produces an adhesive with lower viscosity and higher adhesion.

The silanol residue functionality in (III) can be replaced by a trimethylsiloxane group by means of a trimethylsilylation reaction to give a silicone PSA (IV) as shown in said Figure 11, which hence shows lesser tendency to react further or to form hydrogen bonds with active principles having amino functional groups.

Using this type of technology Dow Coming produces two silicone PSA types: standard Silicone Bio-PSA®, and Silicone Bio-PSA® compatible with amino groups.

Each of these types is available in 3 different degrees of viscosity, plasticity, and adhesion.

The following Table 1 shows the characteristics of said products.

**Table 1**

| Silicone PSA | Nomenclature | Resin (II)/ polymer(I) | Viscosity (g)* | Adhesion (g/cm)* | Plasticity** (100xcm) |
|---|---|---|---|---|---|
| Bio-PSA^{®} Standard | 7-4400 | 65/35 | <70, | 800 | 64 |
| | (III-a) | | | | |
| | 7-4500 | 60/40 | 70 | 600 | 41 |
| | (III-b) | | | | |
| | 7-4600 | 55/45 | 500 | 400 | 22 |
| | (III-c) | | | | |
| Bio-PSA^{®} Amine-Compatible | 7-4100 | 65/35 | 70 | 750 | 51 |
| | (lV-a) | | | | |
| | 7-4200 | 60/40 | 150 | 650 | 33 |
| | (lV-b) | | | | |
| | 7-4300 | 55/45 | 500 | 500 | 26 |
| | (lV-c) | | | | |

Said silicone PSAs are available commercially in the form of solutions in solvents chosen from heptane (commercial code 01), ethyl acetate (commercial code 02), toluene (commercial code 03) and typically at concentrations of 60% by weight.

It is recognized that the silicone PSAs are particularly suitable for transdermal systems in that they satisfy said requirements.

Because of their molecular structure, silicone PSAs present the following properties:
low surface energy, low glass transition temperature, high degree of flexibility, good adhesiveness and cohesion force, high permeability to a wide range of therapeutic agents.

Again, in contrast to other acrylic based PSAs and synthetic rubber, they are free of plasticizers, catalysts or other potentially toxic agents.

Silicone PSAs are particularly effective when used to prepare the third aforedescribed type of transdermal patch, i.e. patches in which the active principle is dispersed in the adhesive matrix, in that in addition to said advantages they enable greater diffusion of the active principle in the skin.

However the active principle, present in supersaturation concentrations in these types of adhesive, tends to crystallize with the passage of time, consequently the patch presents lesser therapeutic effectiveness in that the active principle able to diffuse into the skin decreases. This is a serious drawback, as this type of patch cannot be stored other than for very short time periods.

In "Eudragits: Role as crystallization inhibitors in drug in adhesive transdermal system of estradiol" European Journal of Pharmaceutics and Biopharmaceutics 52 (2001) 173-180, P.N. Kotiyan et al. have shown that in a transdermal patch of the third type containing estradiol as active principle and in which the adhesive matrix consists of a hexylacrylate-acrylic acid copolymer, containing 5% of active principle dispersed in the adhesive matrix, particular cationic copolymers of acrylic type in micronized form, specifically Eudragit® E PO and Eudragit® RL PO at concentrations between. 0.25-2.0 mg/cm² are able to prevent crystal formation even after time periods of 6 months at ambient temperature.

French patent application FR 2 749 586 A discloses a transdermal patch comprising trigesterone and BIO-PSA 7-4301.

### TECHNICAL PROBLEM

The need was felt for a transdermal patch in which the active principle is dispersed within an adhesive matrix consisting of pressure sensitive silicone adhesives (PSAs) which is storage-stable for prolonged time periods.

### SUMMARY OF THE INVENTION

The Applicant has now unexpectedly found that storage-stable patches can be obtained by adding between 1 and 10% by weight on the silicone PSA weight of one or more copolymers of acrylic and/or methacrylic esters containing amino or salified ammonium groups.

The Applicant has also found that said acrylic and/or methacrylic ester copolymer quantity range is critical in obtaining the desired results. In this respect, less than 1 wt% concentrations of said component do not prevent the formation of crystals of the active principle. At greater than 10% concentrations of said copolymer, a significant alteration occurs in the mechanical and biopharmaceutical properties.

The present invention therefore provides a patch suitable for transdermal or local administration of at least one active principle comprising:
a) a matrix based on pressure sensitive adhesive silicone polymers containing:
   a-1) said active principle in concentrations between 1 and 10% by weight on the total weight of said dry adhesive matrix,
   a-2) said silicone polymers in quantities between 80 and 98% by weight on the total weight of said dry adhesive matrix,
   a-3) at least one copolymer of cationic type of acrylic and/or methacrylic esters containing amino groups or salified ammonium groups in a concentration between 1 and 10% by weight on the total weight of said adhesive silicone polymers.
b) a support layer on which said adhesive matrix (a) is located,
c) a protective layer disposed on said adhesive matrix.

### DESCRIPTION OF THE FIGURES

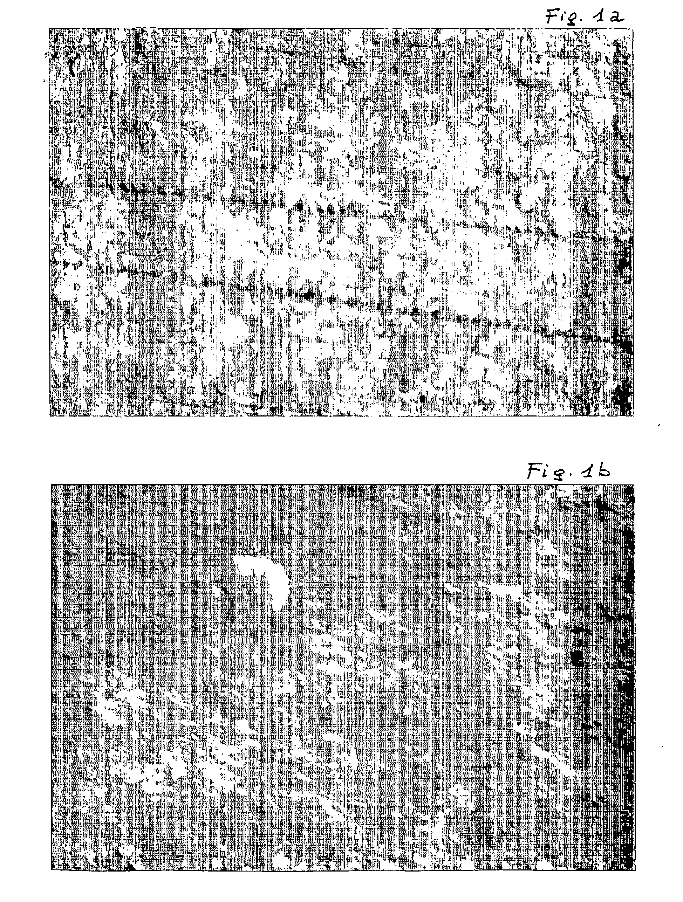

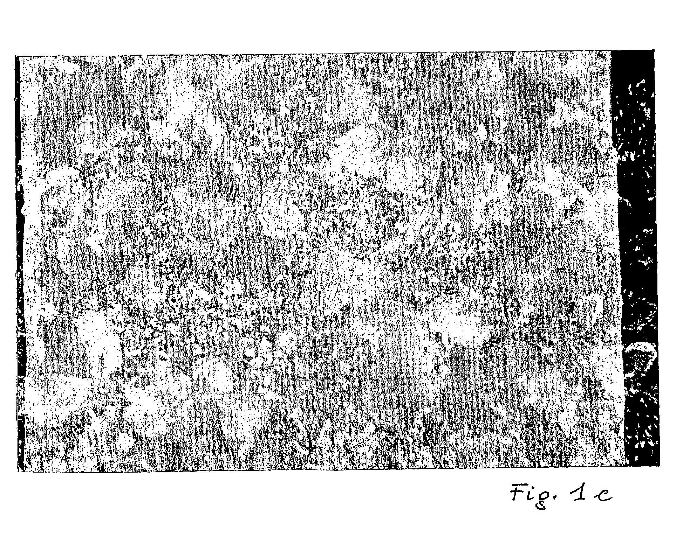

Figure 1a represents an enlarged (10X) photo taken via a ZEISS AXIOLAB® optical microscope with polarized light, of the patch obtained with the formulation 1 as described in Example 1 at time t=0, Figure 1b represents an enlarged (10X) photo of the same patch after storing for 7 days at 20°C and finally Figure 1c represents an enlarged (10X) photo of the same patch after storing for 28 days at 20°C.

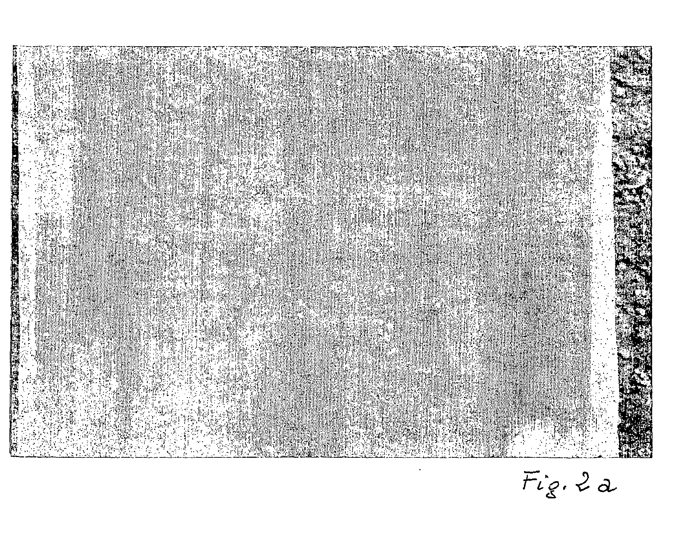

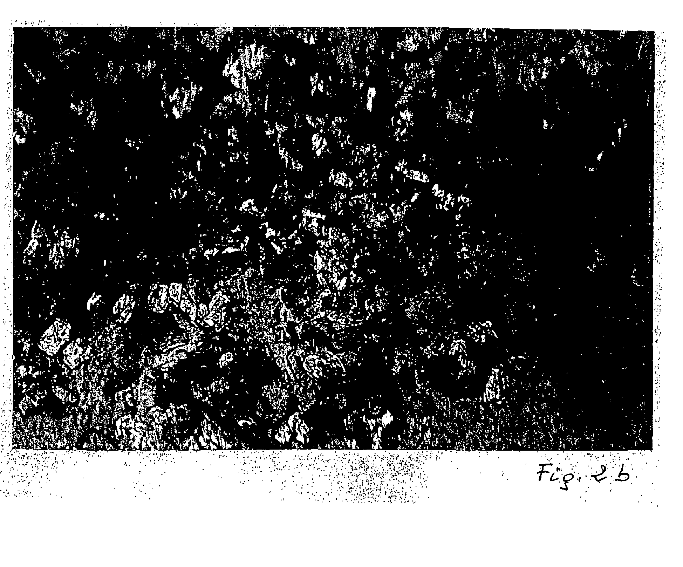

Figure 2a represents an enlarged (10X) photo taken via the same optical microscope with polarized light, of the patch obtained with the formulation 2 described in Example 1 after storing for 7 days at 20°C, Figure 2b represents an enlarged (10X) photo of the patch after storing for 28 days at 20°C.

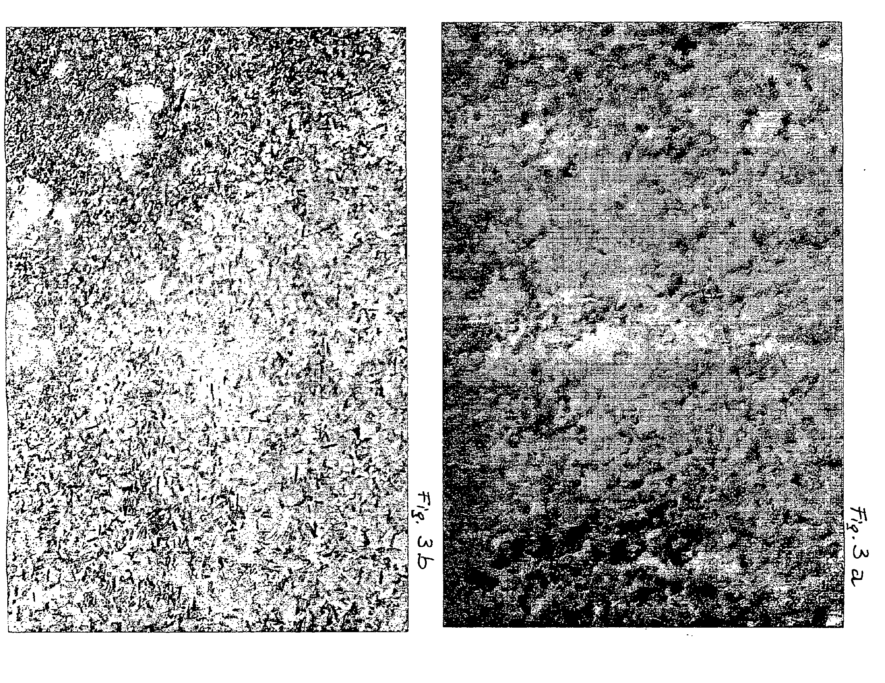

Figure 3a represents an enlarged (10X) photo taken via the same optical microscope with polarized light, of the patch obtained with the formulation 3 as described in Example 1 at time t=0, Figure 3b represents an enlarged (10X) photo of the same transdermal patch after storing for 30 days at 20°C.

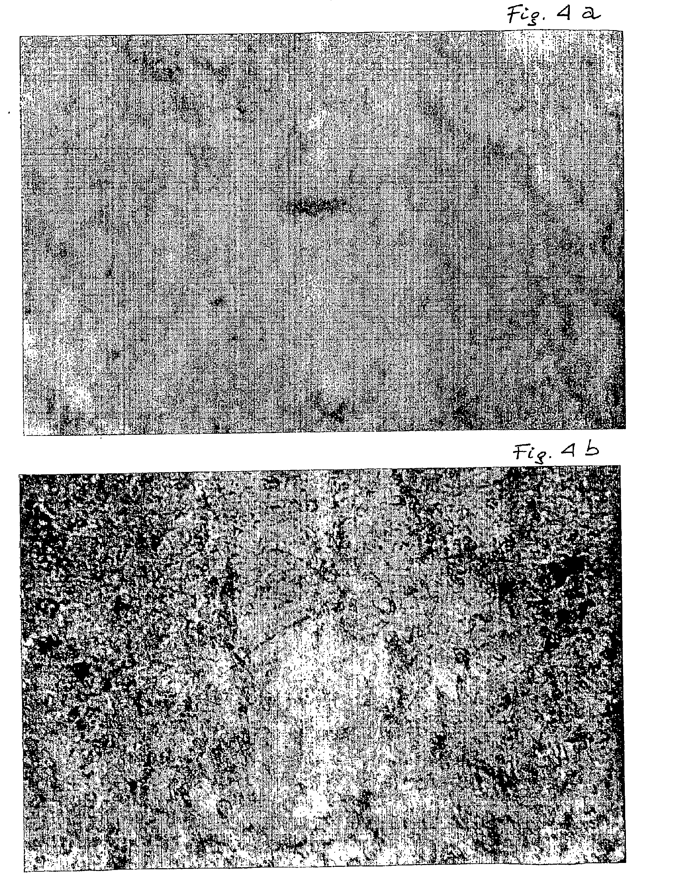

Figure 4a represents an enlarged (10X) photo taken via the same optical microscope with polarized light, of the patch obtained with the formulation 4 at time t=0, Figure 4b represents an enlarged (10X) photo, taken via the same microscope, of the same transdermal patch after storing for 100 days at 20°C.

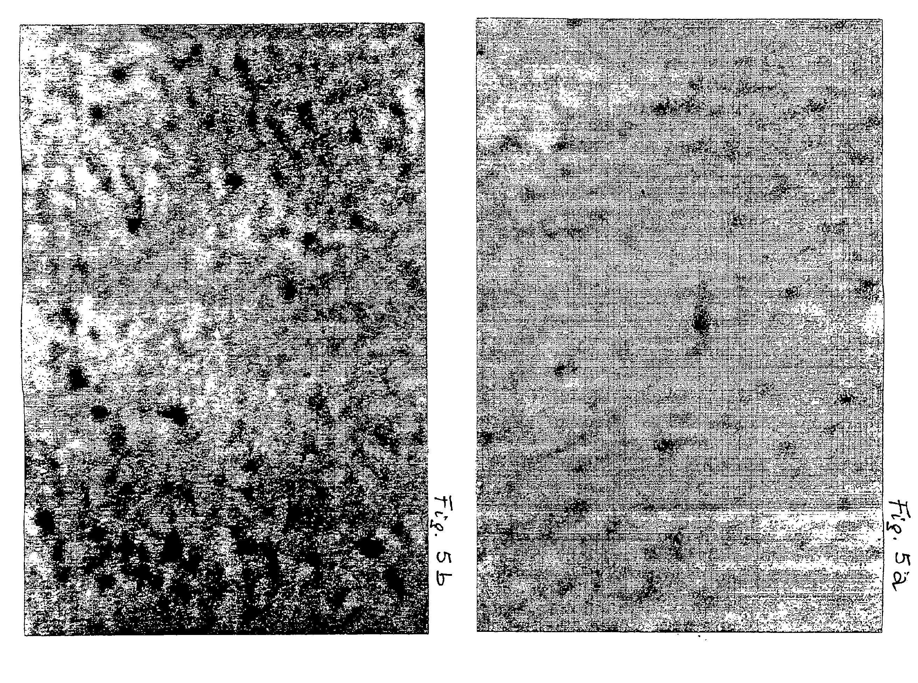

Figure 5a represents an enlarged (10X) photo taken via the same optical microscope, of the patch obtained in example 1with the formulation 5 at time t=0,
Figure 5b represents an enlarged (10X) photo of the same transdermal patch after storing for 100 days at 20°C.

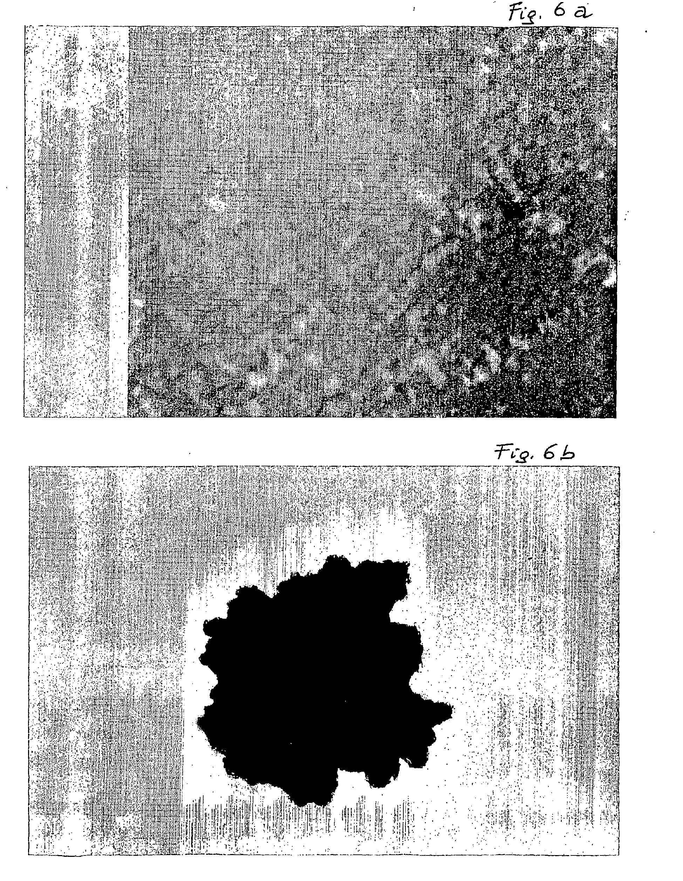

Figure 6a represents an enlarged (10X) photo taken via the same optical microscope with polarized light, of the patch obtained as described in Example 2 at time t=0, with the formulation 4 at t=0. Figure 6b represents an enlarged (10X) photo of the same transdermal patch after storing for 25 days at 20°C.

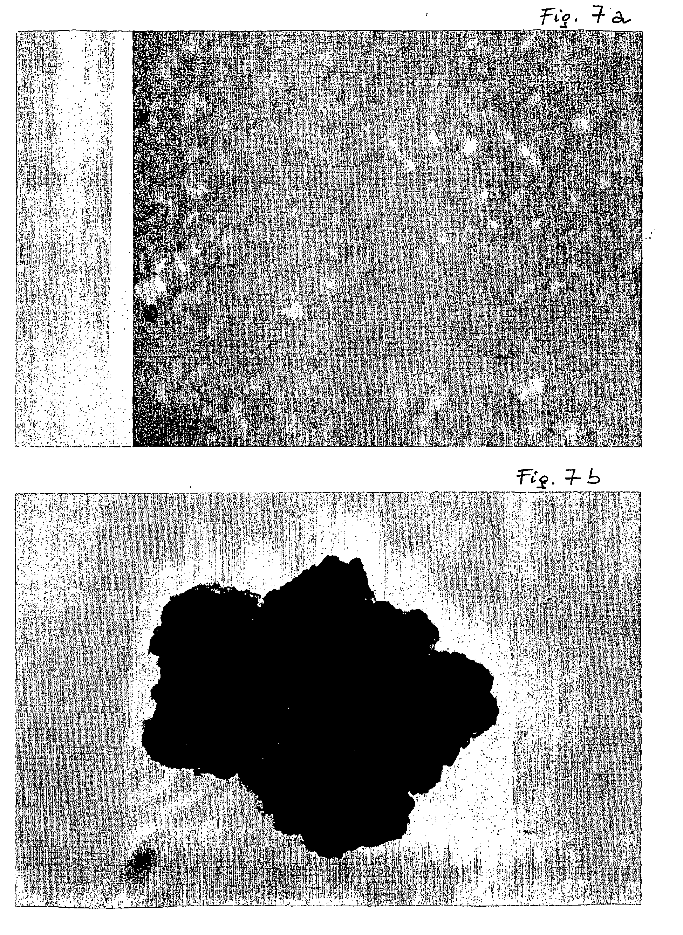

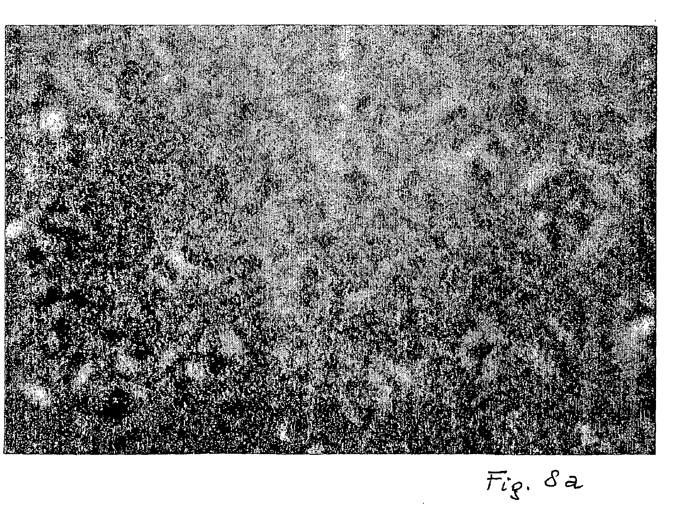

Figure 7a represents an enlarged (10X) photo taken via the same optical microscope with polarized light, of the patch obtained as described in Example 2 with the formulation 6 at time t=0, Figure 7b represents an enlarged (10X) photo of the same transdermal patch after storing for 50 days at 20°C.

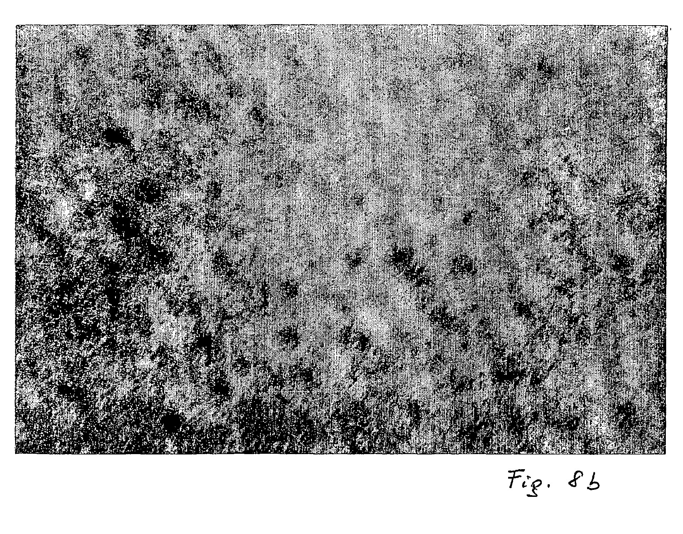

Figure 8a represents an enlarged (10X) photo taken via the same optical microscope with polarized light, of the patch obtained as described in Example 2 with the formulation 7 after storing for 45 days at 20°C, Figure 8b represents an enlarged (10X) photo of the same transdermal patch after storing for 7 months at 20°C.

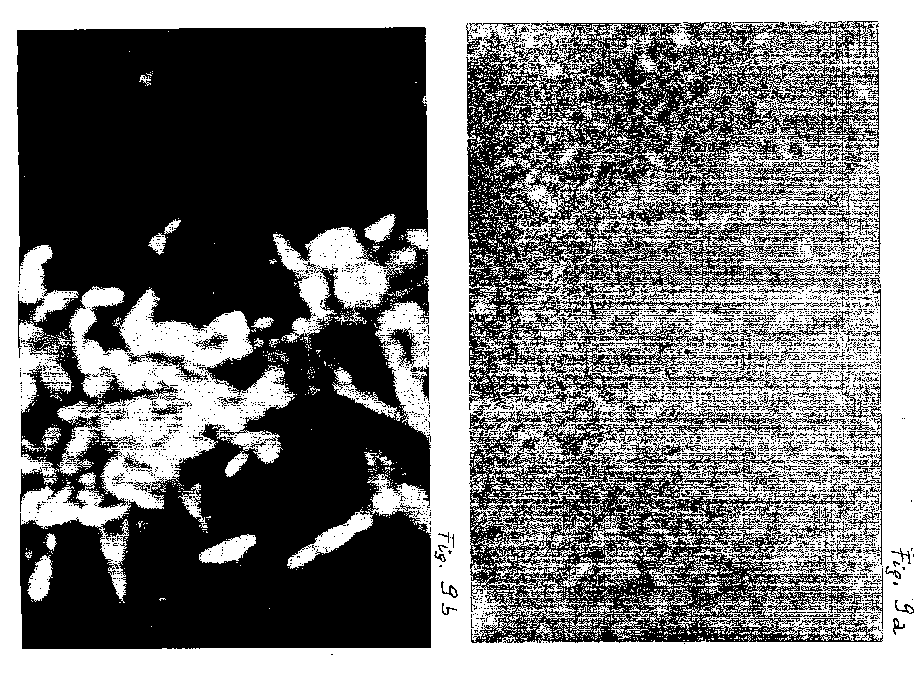

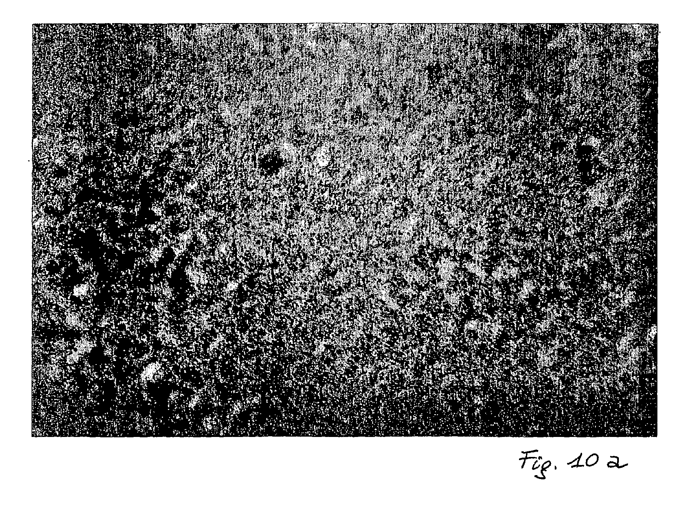

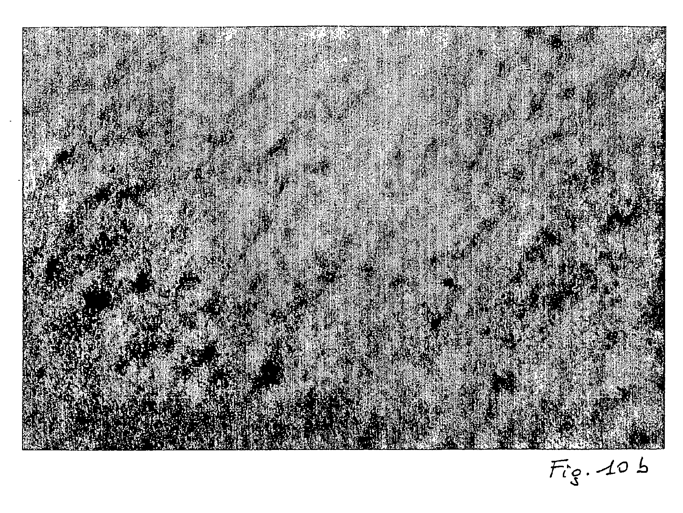

Figure 9a represents an enlarged (10X) photo taken via the same optical microscope with polarized light, of the patch obtained as described in Example 2 with the formulation 8 at time t=0, Figure 9b represents an enlarged (10X) photo of the same transdermal patch after storing for 40 days at 20°C. Figure 10a represents an enlarged (10X) photo taken via the same optical microscope with polarized light, of the patch obtained as described in Example 2 with the formulation 9 after storing for 48 days at 20°C, Figure 10b represents an enlarged (10X) photo of the same transdermal patch after storing for 7 months at 20°C.
Figure 11 represents the synthesis scheme for the silicone PSAs used in the patch of the present invention.

### DETAILED DESCRIPTION OF THE INVENTION

The patch of the present invention can contain any type of active principle compatible with and dissolvable in the matrix.

However this system is particularly preferred for drugs with urinary antispastic activity, a particularly preferred drug pertaining to this class being oxybutynin.

Another class of drugs to which this system is applicable are the drugs used to treat benign prostatic hypertrophy, the active principle terazosin and finasteride being particularly preferred.

Another class of active principles usable in the transdermal patch is that of the steroidal hormones and in particular the estrogens such as dehydroepiandrosterone, estradiol, and the progestinics such as norethisterone.

The following classes of drugs applicable to the patch of the present invention can also be mentioned, such as non-steroidal anti-inflammatories and in particular arylalkanoic acids such as ibuprofen, and oxicams such as piroxicam; the non-selective beta blockers such as propanolol, and the selective beta blockers such as atenolol; calcium antagonists and in particular dihydropyridines such as nifedipine; and benzodiazepines such as clonazepam, triazolam, lorazepam.

When the active principle of the present invention contains amino groups, the silicone polymer is preferably chosen from the amine-compatible Bio-PSAs®, 7-4100 (IV-a), 7-4200 (IV-b), 7-4300 (IV-c), the characteristics of which are reported in Table 1. When the active principle does not present amino groups, a standard Bio-PSA® is preferably used chosen from 7-4400 (III-a), 7-4500 (III-b), 7-4600 (III-c).

Other preferred embodiments comprise the use of mixtures of standard Bio-PSA® with amine-compatible Bio-PSA®, both for active principles containing amino groups and for those not containing them.

The patches of the present invention can also contain a mixture of two or three types of 7-4400 - 7-4600 standard Bio-PSA® polymers or a mixture of two or three types of 7-4100 - 7-4300 amine-compatible Bio-PSA® polymers.

The expert of the art can therefore, on the basis of the type of active principle and the mechanical properties to be obtained, choose which silicone polymers or whether to opt for a mixture of silicone polymers chosen from the aforementioned.

According to a preferred embodiment, use is made of solutions of said silicone PSAs in ethyl acetate or commercial, products containing 60% of polymer (i.e. the commercial standard. Bio-PSA® products 7-4402, 7-4502, 7-4602, and the amine-compatible products 7-4102, 7-4202, 7-4302).

The copolymer(a-3) is preferably chosen from:
i) copolymers of cationic type based on dialkylaminoalkylmethadrylate, and neutral alkylmethacrylate esters, where alkyl means a C₁-C₁₀ linear or branched alkyl residue, said copolymers having an average molecular weight between . 100,000. and 500,000 and in which the ratio of repetitive dialkylaminoalkylmethacrylate/neutral ester units is between 2:1 and 1:2;
ii) copolymers of cationic type based on trialkylammoniumalkylmethacrylate, and neutral alkylmethacrylate esters, neutral alkylacrylate esters, where alkyl means a C₁-C₁₀ linear or branched alkyl residue, said copolymers having an average molecular weight between 100,000 and 500,000 and in which the alkylmethacrylate and methylmethacrylate/trialkylammoniumalkylmethacrylate ratio is between 40:1 and 20:1;
iii) mixtures of the copolymers (I) and (ii).

According to a particularly preferred embodiment the copolymer is chosen from the group consisting of:
a-3-1) poly-(butylmethacrylate), (2-dimethylaminoethyl)-methacrylate, methylmethacrylate) in which the ratio of said 3 monomers is respectively 1:2:1, and is characterised by an average molecular weight of 150,000. This product is available commercially with the brand name of Eudragit® E100,
a-3-2) poly(ethylacrylate, methylmethacrylate, trimethylammoniumethytmethacrytate chloride) characterised by an average molecular weight of 150,000 and in which the ratio of said monomers is 1:2:0.2. This product is available commercially with the brand name of Eudragit® RL 100,
a-3-3) poly(ethylacrylate, methylmethacrylate, trimethylammoniumethylmethacrylate chloride) characterised by an average molecular weight of 150,000 and in which the ratio of said monomers is 1:2:0.1. This product is available commercially with the brand name of Eudragit® RS100,
a-3-4) mixtures of two or all the copolymers a-3-1), a-3-2), a-3-3).

Said copolymers are preferably added during the preparation of the adhesive patch matrix in the form of an ethyl acetate solution which contains them in a quantity of about 10%.

The acrylic copolymer (a-3-1 ) can be used when the adhesive matrix contains either an active principle of basic type or an active principle of acid, alcohol or other type.

Eudragit RS100 (a-3-3) is preferably used when the active principle is of basic type, and Eudragit RL 100 (a-3-2) when the active principle is of acid type.

The adhesive matrix can contain other additives such as solubilizing agents, for example polypropylene glycol, polyethylene glycols of various molecular weights, glycerol, and absorption enhancers such as caprolactone, unsaturated fatty acids and their esters, and terpenes.

The support layer can be chosen from any one of those habitually used for transdermal patches. If necessary a film permeable to water vapour can be used to prevent maceration of the skin, such as that available commercially with the brand name Cotran® 97.15 from 3M.

The protective film (c) used must be a non-siliconized film such as that available commercially with the brand name Scotch Pack® 1022 from 3M.

The patches of the present invention are prepared by a process comprising the following stages:
α) adding the solution of polymer of acrylic and/or methacrylic esters of cationic type containing amino groups or salified ammonium groups (a3) in an organic solvent, preferably ethyl acetate, to the solution of silicone PSA in the same organic solvent used for (a3), preferably ethyl acetate,
β) adding the active principle to the mixture obtained in the preceding stage (α), and keeping the resultant mixture under stirring for 3 hours,
γ) spreading on the support (b) the mixture coming from the preceding stage (β), drying it and applying the protective sheet (c) with conventional machines.

Some examples of the preparation of the patch of the present invention are provided for illustrative but not limitative purposes.

### EXAMPLE 1

### 1-A Preparation of the polymer solutions used for preparing the matrix

### Composition:

| **Form. no** | Oxybutynin base (**g**) | **BIO-PSA 7-4302(g)** | **BIO-PSA 7-4202 (g**) | **Propylene glycol (g)** | **Eu. E* (g)** | **Eu. RL* (g)** | **Eu. RS* (g)** | **Prep. date** |
|---|---|---|---|---|---|---|---|---|
| **1** | 2.58 | 97.42 | - | - | - | - | - | 05/07/01 |
| **2** | 2.35 | 88.61 | - | 2.98 | 6.06 | - | - | 05/07/01 |
| **3** | 2.10 | 79.10 | - | 2.66 | 16.14 | - | - | 11/06/01 |
| **4** | 1.83 | 81.53 | - | - | 16.64 | - | - | 28/09/01 |
| **5** | 1.83 | 81.53 | - | - | - | - | 16.64 | 28/09/01 |
| **6** | 1.83 | 81.53 | - | - | - | 16.64 - | | 28/09/01 |
| **7** | 1.57 | 69.90 | - | - | 28.53 | | - | 01/10/01 |
| **8** | 1.57 | 69.90 - | - | - | - | - | 28.53 | 01/10/01 |
| **9** | 1.75 | 77.96 | - | - | 20.29 - | - | - | 03/10/01 |
| **10** | 1.75 | 77.96 | - | - | - | - | 20.29 | 03/10/01 |
| **11** | 1.65 | 73.57 | - | - | 24.77 | - | - | 03/10/01 |
| **12** | 1.65 | 73.57 | - | - | - | - | 24.77 | 03/10/01 |
| **13** | 2.44 | - | 97.56 | | | | | 28/05/01 |
| **14** | 2.20 | - | 81.50 | | | | 1.6.30 | 11 /06/01 |
| **15** | 2.10 | - | 79.10 | 2.66 | | | 16.14 | 11/06/01 |
| **16** | 2.10 | - | 79.10 | 2.66 | 16.14 | | | 11/06/01 |
| **17** | 3.59 | 64.90 | 28.34 | | | | 3.17 | 30/11 /01 |

| | | | | | | | | |
|---|---|---|---|---|---|---|---|---|
| *Polymer solution of Eudragit E 100, RL 100 or RS 100 10% m/m in ethyl acetate | | | | | | | | |

The formulations were prepared by adding the Eudragit® solution and, if necessary, the propylene glycol to the Bio-PSA 7-4302 maintained under stirring. The oxybutynin was added to the mixture obtained, maintaining the system under stirring for 3 hours.

The polymer system obtained was left to under rest until complete removal of air, before being used.

### 1-B Patch preparation

Support: Cotran® 97.15 (3M);
Protective sheet: Scotchpack® 1022 (3M)

The matrix was spread on the protective sheet and dried using the Matis spreading machine, model LTE-S (M).

| | |
|---|---|
| Spreading rate | 1 m/min; |
| Drying time | 20 min; |
| Drying temperature | 50° C |
| Distance blade-protective sheet | 350 µm |

On termination of the process the patch obtained was packaged in air-impermeable envelopes and stored at 20°C.

### 1-C Oxybutynin content

The oxybutynin content was determined by dissolving a 2.54 cm² sample in a suitable volume of ethyl acetate and determining the content by HPLC-UV.

### 1-D Monitoring of crystal formation

Formation of oxybutynin crystals was monitored on a 144 cm² surface by optical microscope. The patches were checked immediately after preparation and then once a week. Any matrix changes were recorded by photographing with 10x magnification a patch sample made to adhere to a glass slide.

### 1-E Study of cutaneous permeability

The in vitro permeability studies were conducted by the modified Franz-type diffusion cell method (P. Minghetti, J. Pharm. and Pharmacol., 51(6): 729-734, 1999), using as membrane human epidermis from one and the same donor. The oxybutynin quantity which had permeated was determined_{.} by HPLC-UV. The results are the mean of three determinations.

### 1-F Adhesiveness

The capacity of the patches to adhere to the skin was evaluated by the Thumb Tack Test (D. Satas "Tack" in Handbook of Pressure Sensitive Technology edited by Donatas Satas,1999: Minghetti et al. Drug Dev. Ind. Pharm., 25(1): 1-6,1999).

### 1-G Results

### Monitoring of crystal formation

### Oxybutynin recrystallization time

| Form. | Days |
|---|---|
| 1 | 7 |
| 2 | 19 |
| 3 | 45 |
| 4 | >360 |
| 5 | >360 |
| 6 | 24 |
| 7 | >360 |
| 8 | >360 |
| 9 | >360 |
| 10 | >360 |
| 11 | >360 |
| 12 | >360 |
| 13 | 90 |
| 14 | 180 |
| 15 | 150 |
| 16 | 150 |
| 17 | >360 |

### 1-H Oxybutynin content and cutaneous permeability

Oxybutynin content in the patches and quantity permeated in 24 h

| Form. | Content (µg/cm²) | quantity permeated in 24h |
|---|---|---|
| | | (µg/cm²) |
| 4 | 355±14 | 120.01 ±.20.71 |
| 5 | 329±21 | 110:38 ± 23.12 |
| 6 | 381 ±9 | 90.01 ±28.38 |
| 12 | 368±15 | 103.89 ± 39.24 |

### EXAMPLE 2

### 2-A Preparation of the polymer solutions used for preparing the matrix

### Composition:

| **Form. No.** | **lbu (g) (g)** | **Bio- psa 4202(g)** | **Bio- psa 4302(g)** | **Bio- psa 4502(g)** | **Bio- psa 4602(g)** | **Prop. glycol** | **Eu E* (g)** | **Eu RS* (g)** | **Eu RL* (g)** | **Prep.date.** |
|---|---|---|---|---|---|---|---|---|---|---|
| 1 | 1.96 | 98.04 - | - | - | - | - | - | - | - | 06/03/01 |
| 2 | 1.96 | - | 98.04 | | - | - | - | - | - | 24/01/01 |
| 3 | 1.96 | | - | 98.04 - | | - | - | - | - | 06/03/01 |
| 4 | 1.96 | - | - | - | 98.04 - | | - | - | - | 06/03/01 |
| 5 | 1.91 | - | 95.69 - | - | - | - | 2.39 | - | - | 01/03/01 |
| 6 | 1.9.1 | - | - | - | 95.69 | 2.39 | - | - | - | 10/04/01 |
| 7 | 1.63 | - | | | 81.63 | 2.04 | 14.69 | - | - | 14/06/01 |
| 8 | 1.63 | - | - | - | 81.63 | 2.04 | - | 14.69 | - | 14/06/01 |
| 9 | 1.63 | - | - | - | 81.63 | 2.04 | - | - | 14.69 | 13/06/01 |

| | | | | | | | | | | |
|---|---|---|---|---|---|---|---|---|---|---|
| *Polymer solution of Eudragit E 100, RL 100 or RS 100 10% m/m in ethyl acetate | | | | | | | | | | |

The formulations were prepared by adding the Eudragit® solution and, if necessary, the propylene glycol to the appropriate BIO PSA maintained under stirring.

The ibuprofen was added to the mixture obtained, maintaining the system under stirring for 3 hours.

The polymer system obtained was left under rest until complete removal of air, before being used.

### 2-B Patch preparation

Support: Scotchpak 1022 (3M);
Protective sheet: Scotchpack 1022 (3M)

The matrix was spread on the protective sheet and dried using the Matis spreading machine, model LTE-S (M).

| | |
|---|---|
| Spreading rate | 1 m/min; |
| Drying time | 20 min; |
| Drying temperature | 50° C |
| Distance spreader blade-protective sheet | 300 µm |

On termination of the process the patch obtained was packaged in air-impermeable envelopes and stored at 4°C and 20°C.

### 2-C Ibuprofen content

The ibuprofen content was determined by dissolving a 2.54 cm² sample in a suitable volume of ethyl acetate and determining the content by HPLC-UV.

### 2-D Monitoring of crystal formation

Formation of ibuprofen crystals was monitored on a 144 cm² surface by optical microscope. The patches were checked immediately after preparation and then once a week. Any matrix changes were recorded by photographing with 10x magnification a patch sample made to adhere to a glass slide.

### 2-E Study of cutaneous permeability

The in vitro permeability studies were conducted by the modified Franz-type diffusion cell method (P. Minghetti, J. Pharm. and Pharmacol., 51 (6): 729-734, 1999), using human epidermis as membrane from one and the same donor. The ibuprofen quantity which had permeated was determined by HPLC-UV. The results are the mean of three determinations.

### 2-F Adhesiveness

The capacity of the patches to adhere to the skin was evaluated by the Thumb Tack Test (D. Satas "Tack" in Handbook of Pressure Sensitive Technology edited by Donatas Satas, 1999: Minghetti et al. Drug Dev. Ind. Pharm., 25(1): 1-6, 1999).

### 2-G Results

### Ibuprofen content e quantity permeated in 24 h

| Form. | Content (µg/cm²) | quantity permeated in 24h |
|---|---|---|
| | | (µg/cm²) |
| 6 | 368±15 | 109±13 |
| 7 | 323±23 | 117±4 |
| 9 | 338±10 | 145 ± 25 |

### Days before appearance of crystals

| Form. | Temperature | Days |
|---|---|---|
| 1 | 20°C | 1 |
| 2 | 20°C | 5 |
| 3 | 20°C | 25 |
| 4 | 20°C | 25 |
| 5 | 20°C | 5 |
| 6 | 20°C | 50 |
| 7 | 20°C | > 21 months |
| 7 | 4°C | > 21 months |
| 7 | 40°C | > 12 months |
| 8 | 20°C | 40 |
| 9 | 20°C | > 21 months |
| 9 | 4°C | > 21 months |
| 9 | 40°C | > 12 months |

### EXAMPLE 3

### Patches containing nifedipine (NIF)

### 3-A Preparation of the polymer solutions used for preparing the matrix Composition:

| **Form. No.** | **NIF (g)** | **Bio-psa 4202 (g)** | **Bio-psa 4302 (g)** | **Eu E* (g)** | **Eu RL* (g)** | **Eu RS* (g)** | **Prep. Date** |
|---|---|---|---|---|---|---|---|
| 1 | 1.96 | 98.04 | | | | | 06/03/2002 |
| 2 | 1.64 | 81.97 | | 16.39 | | | 06/03/2002 |
| 3 | 1.64 | 81.97 | | | 16.39 | | 06/03/2002 |
| 4 | 1.64 | 81.97 | | | | 16.39 | 06/03/2002 |

| | | | | | | | |
|---|---|---|---|---|---|---|---|
| *Polymer solution of Eudragit E 100, RL 100 or RS 100 10% m/m in acetone | | | | | | | |

The formulations were prepared by dissolving nifedipine in the Eudragit solution and adding the solution obtained to the appropriate BIO PSA maintained under stirring. The mixture obtained was maintained under stirring for 3 hours. The polymer system obtained was left under rest until complete removal of air, before being used.

### 3-B Patch preparation

Support: Scotchpak 1022 (3M);
Protective sheet: Scotchpack 1022 (3M)

The matrix was spread on the protective sheet and dried using the Matis spreading machine, model LTE-S (M).

| | |
|---|---|
| Spreading rate | 1 m/min; |
| Drying time | 20 min; |
| Drying temperature | 50° C |
| Distance blade-protective sheet | 350 µm |

On termination of the process the patch obtained was packaged in air-impermeable envelopes and stored at 4°C and 20°C.

### 3-D Monitoring of crystal formation

Formation of nifedipine crystals was monitored on a 144 cm² surface by optical microscope. The patches were checked immediately after preparation and then once a week. Any matrix changes were recorded by photographing with 10x magnification a patch sample made to adhere to a glass slide.

### 3-E Results

### Days before appearance of crystals

| Form. | Temperature | Days |
|---|---|---|
| 1 | 20°C | 7 |
| 2 | 20°C | >10 months |
| 3 | 20°C | >10 months |
| 4 | 20°C | >10 months |

### EXAMPLE 4

Patches containing dehydroepiandrosterone (DHEA)

### 4-A Preparation of the polymer solutions used for preparing the matrix

### Composition:

| **Form. no.** | **DHEA (g)** | **Bio-psa 4602 (g)** | **isopropanol** | **Eu E (g)** | **Eu RL (g)** | **Eu RS (g)** | **Prep date** |
|---|---|---|---|---|---|---|---|
| 1 | 1.67 | 81.67 | 16.66 | - | - | - | 28/01/2002 |
| 2 | 1.64 | 81.97 | - | - | - | 16.39* | 13/12/2001 |
| 3 | 1.64 | 81.97 | - | 16.39* | - | - | 13/12/2001 |
| 4 | 2.04 | 81.63 | - | 16.33** | - | - | 28/01/2002 |
| 5 | 1.43 | 71.43 | 12.85 | - | 14.29* | - | 25/02/2002 |
| 6 | 1.43 | 71.43 | 12.85 | - | - | 14.29* | 25/02/2002 |

| | | | | | | | |
|---|---|---|---|---|---|---|---|
| *Polymer solution of Eudragit E 100, RL 100 or RS 100 10% m/m in ethyl acetate **Polymer solution of Eudragit E 100 in isopropanol | | | | | | | |

Formulation No. 1 was prepared by dissolving the DHEA in isopropanol and adding the solution obtained to the BIO PSA 4602 maintained under stirring. The system obtained was maintained under stirring for 3 hours.

Formulation No. 2 was prepared by dispersing the DHEA in the BIO-PSA and adding the Eudragit solution. The system obtained was maintained under stirring for 3 hours.

Formulations Nos. 3, 4 were prepared by dissolving the DHEA in the Eudragit solution and adding the solution obtained to the BIO PSA 4602 maintained under stirring. The mixture obtained was maintained under stirring for 3 hours.

Formulations Nos. 5, 6 were prepared by dissolving the DHEA in isopropanol. The final polymer system was obtained by adding the solution obtained to that of Eudragit in the BIO-PSA. The mixture obtained was maintained under stirring for 3 hours.

All the polymer systems obtained were left under rest until complete removal of air, before being used.

### 4-B Patch preparation

Support: Scotchpak 1.022 (3M);
Protective sheet: Scotchpack 1022 (3M)

The matrix was spread on the protective sheet and dried using the Matis spreading machine, model LTE-S (M).

| | |
|---|---|
| Spreading rate | 1 m/min; |
| Drying time | 15 min; |
| Drying temperature | 50° C |
| Distance blade-protective sheet | 350µm |

On termination of the process the patch obtained was packaged in air-impermeable envelopes and stored at 4°C and 20°C.

### 4-C Monitoring of crystal formation

Formation of dehydroepiandrosterone crystals was monitored on a 144 cm² surface by optical microscope. The patches were checked immediately after preparation and then once a week. Any matrix changes were recorded by photographing with 10x magnification a patch sample made to adhere to a glass slide.

### 4-D Results

### Days before appearance of crystals

| | | |
|---|---|---|
| Form. | Temperature | Days |
| 1 | 20°C | 7 |
| 2 | 20°C | >1 year |
| 3 | 20°C | >1 year |
| 4 | 20°C | >1 year |
| 5 | 20°C | >10 months |
| 6 | 20°C | >10 months |

### EXAMPLE 5

### Percutaneous absorption of patches containing oxybutynin after single and multiple dose in healthy male volunteers

The object of this pilot study is to evaluate the in vivo absorption, kinetic profile and adhesiveness of the 36 cm² patch containing oxybutynin based on formulations No. 17. (patch A), No. 8 (patch B) and No. 14 (patch C) of the example.

### Method

Single and multiple application, pharmacokinetic pilot studies on the same subject in three successive phases.

### Number of subjects (programmed and analyzed)

Three subjects programmed, three subjects analyzed. Inclusion criteria

Sex: males, aged 18-45 years, good healthy state, no allergic form, low alcohol, tobacco and caffeine consumption.

### Duration of treatment

Phase I and II single dose, phase III three applications for 3 days

### LIST OF ABBREVIATIONS AND DEFINITION OF TERMS

- ANOVA: analysis of variance
- AUCₜ: area below concentration/time curve
- Cₘₐₓ: maximum plasma concentration
- CV: variance coefficient
- Tₘₐₓ: time for attaining Cₘₐₓ
- t_{½}: half life
- Cₛₛₘₐₓ: maximum concentration in the steady state
- tₛₛₘₐₓ: time for attaining maximum concentration in the steady state
- AUCₛₛ: area under steady state concentration/time curve
- Cₘₑₐₙ: mean concentration
- Cₛₛₘᵢₙ: minimum concentration in the steady state

### EVALUATION CRITERIA (KINETIC)

ln order to evaluate the pharmacokinetic profile of the three formulations, a blood sample was taken before application and after 1, 2, 4, 6, 8, 10, 12, 16, 24, 28, 30, 32, 36, 40 and 48 hours for patches A and B. In the case of patch C the sample was taken before application and after 1, 2, 4, 6, 8,10, 12, 16, 24, 28, 30, 32, 36, 40, 48, 50, 52, 54, 56, 60, 64, 72 hours. The oxybutynin and its main metabolite desethyloxybutynin were determined in plasma. The main kinetic parameters measured and/or calculated were: Cₘₐₓ, tₘₐₓ, AUCₜ for formulations A and B; Cₛₛₘₐₓ, tₛₛₘₐₓ, AUCₛₛ and Cₘₑₐₙ for formulation C.

### Statistical methods

The data and the measured parameters are described using classical statistics: mean, SD, CV%, minimum and maximum values.

The calculated values of AUC₀₋₂₄ₕ and Cₘₐₓ in plasma for oxybutynin and desethyloxybutynin after administering the patches A, B and C were compared by variance analysis (ANOVA) with a significance level p < 0.05.

### Results

### Formulation A

The concentration peaks were: tₘₐₓ =23.33± 13.01, Cₘₐₓ =0.47 ± 0.20 ng/mL.

The half life was 24.15 ± 20.90 h and MRT. 44.74 ± 28.46 h, the AUCₜ was 20.76 ± 0.78ng*h/mL.

### Formulation B

The concentration peaks. (Cₘₐₓ = 0.69 ± 0.29 ng/mL) were attained after 20 ± 6.93 h, the AUCₜ was 16.57 ± 5.70 ng*h/mL, half life 15.40 ± 5.40 h and MRT 33.07 ± 4.34h.

### Formulation C

In the steady state Cₛₛₘᵢₙ was 0.30 ± 0.17 ng/mL and Cₛₛₘₐₓ 0.61 ± 0.27 with Cₘₑₐₙ 0.48 ±0.22 ng/mL and 69.72 ± 11:96 as %PTF. The AUCₛₛ was 11.42 ± 5.33 ng*h/mL.

To compare the three formulation studies, the Shapiro-Wilk test for normal distribution was carried out for Cₘₐₓ (p = 0.976) and for AUC₀₋₂₄ₕ (p = 0.711 ), indicating no statistical difference between them.

The variance analysis was carried out using ANOVA, and provided the same results: for Cₘₐₓ (p = 0.104) and for AUC₀₋₂₄ₕ (p = 0.082).

### Conclusions

The patches A, B and C reached satisfactory plasma concentrations of oxybutynin and desethyloxybutynin within the 0-24 hour application range. Absorption seems qualitatively to be fairly rapid and protracted, so ensuring pharmacological activity with a single daily application.

## Claims

1. A patch suitable for transdermal or local administration of at least one active principle comprising:
a) a matrix based on pressure sensitive adhesive silicone polymers containing:
a-1) said active principle in concentrations between 1 and 10% by weight on the total weight of said dry adhesive matrix,
a-2) said silicone polymers in quantities between 80 and 98% by weight on the total weight of said dry adhesive matrix,
a-3) at least one copolymer of cationic type of acrylic and/or methacrylic esters containing amino groups or salified ammonium groups in a concentration between 1 and 10% by weight on the total weight of said adhesive silicone polymers.
b) a support layer on which said adhesive matrix (a) is located,
c) a protective layer disposed on said adhesive matrix.

2. The patch as claimed in claim 1, **characterised in that** the active principle is chosen from the class consisting of drugs with urinary antispastic activity, drugs used for treating prostatic hypertrophy, steroidal hormones, steroidal anti-inflammatories, non-selective and selective beta blockers, calcium antagonists, benzodiazepines.

3. The patch as claimed in claim 2, **characterised by** containing oxybutynin as active principle.

4. The patch as claimed in claim 2, **characterised by** containing an active principle chosen from terazosin and finasteride.

5. The patch as claimed in claim 2, **characterised by** containing an active principle chosen from dehydroepiandrosterone and estradiol

6. The patch as claimed in claim 2, **characterised by** containing norethisterone as active principle.

7. The patch as claimed in claim 2, **characterised by** containing ibuprofen as active principle.

8. The patch as claimed in claim 2, **characterised by** containing piroxicam as active principle.

9. The patch as claimed in claim 2, **characterised by** containing propanolol as active principle.

10. The patch as claimed in claim 2, **characterised by** containing at atenolol as active principle.

11. The patch as claimed in claim 2, **characterised by** containing nifedipine as active principle.

12. The patch as claimed in claim 2, **characterised by** containing clonazepam as active principle.

13. The patch as claimed in claim 2, **characterised by** containing triazolam as active principle.

14. The patch as claimed in claim 2, **characterised by** containing lorazepam as active principle.

15. The patch as claimed in any one of claims 1-14, **characterised in that** the copolymer (a-3) is chosen from the group consisting of:
i) copolymers of cationic type based on dialkylaminoalkylmethacrylate, and neutral alkylmethacrylate esters, where alkyl means a C₁-C₁₀ linear or branched alkyl residue, said copolymers having an average molecular weight between 100,000 and 500,000 and in which the ratio of repetitive dialkylaminoalkylmethacrylate/neutral ester units is between 2:1 and 1:2;
ii) copolymers of cationic type based on trialkylammoniumalkylmethacrylate, and neutral alkylmethacrylate esters, neutral alkylacrylate esters, where alkyl means a C₁-C₁₀ linear or branched alkyl residue, said copolymers having an average molecular weight between 100,000 and 500,000 and in which the alkylmethacrylate and methylmethacrylate/trialkylammoniumalkylmethacrylate ratio is between 40:1 and 20:1;
iii) mixtures of (i) and (ii).

16. The patch as claimed in claim 15, **characterised in that** the copolymer is chosen from the group consisting of:
a-3-1) poly-(butylmethacrylate, (2-dimethylamino)-methacrylate, methylmethacrylate) in which the ratio of said 3 monomers is respectively 1:2:1, and is **characterised by** an average molecular weight of 150,000;
a-3-2) poly(ethylacrylate, methylmethacrylate, trimethylammoniumethyl-, methacrylate chloride) **characterised by** an average molecular weight of 150,000 and in which the ratio of said monomers is 1:2:0.2;
a-3-3) poly(ethylacrylate, methylmethacrylate, trimethylammoniumethylmethacrylate chloride) **characterised by** an average molecular weight of 150,000 and in which the ratio of said monomers is 1:2:0.1; and
a-3-4) mixtures of two or all the copolymers a-3-1), a-3-2), a-3-3).

17. The patch as claimed in claim 16, **characterised in that** if the active principle is of basic type, said patch contains the component (a-3-3).

18. The patch as claimed in claim 16, **characterised in that** if the active principle is of acid type, said patch contains the component (a-3-2).

19. A process for preparing the patch claimed in any one of claims 1-18, comprising the following stages:
α) adding the solution of the cationic polymer of acrylic and/or methacrylic esters containing amino groups or salified ammonium groups (a3) in an organic solvent to the solution of silicone PSA in the same organic solvent used for (a3),
β) adding the active principle to the mixture obtained in the preceding stage (α), and keeping the resultant mixture under stirring for 3 hours,
γ) spreading on the support (b) the mixture coming from the preceding stage (β), drying it and applying the protective sheet (c) with conventional machines.

20. The process as claimed in claim 19, **characterised by** using solutions of silicone polymers in ethyl acetate, in which case the organic solvent of stage (α) is ethyl acetate.

21. The process as claimed in claim 20, **characterised in that** said solutions of silicone polymers in ethyl acetate contain said polymer in concentrations of 60% by weight on the total weight of said solution.

## Patentansprüche

1. Pflaster, das für die transdermale oder lokale Verabreichung von mindestens einem Wirkstoff geeignet ist, umfassend:
a) eine Matrix, basierend auf druckempfindlichen haftenden Siliconpolymeren enthaltend:
a-1) den Wirkstoff in Konzentrationen zwischen 1 und 10 Gew.% zum Gesamtgewicht der trockenen haftenden Matrix,
a-2) die Siliconpolymere in Mengen zwischen 80 und 98 Gew.% zum Gesamtgewicht der trockenen haftenden Matrix,
a-3) mindestens ein Copolymer vom kationischen Typ von Acryl- und/oder Methacrylestern, enthaltend Aminogruppen oder versalzte Ammoniumgruppen in einer Konzentration zwischen 1 und 10 Gew.% zum Gesamtgewicht der haftenden Siliconpolymere,
b) eine Stützschicht, auf der die haftende Matrix (a) lokalisiert ist,
c) eine Schutzschicht, angeordnet auf der haftenden Matrix.

2. Pflaster gemäß Anspruch 1, **dadurch gekennzeichnet, daß** der Wirkstoff aus der Klasse ausgewählt wird, bestehend aus Arzneimitteln mit urinär-antispastischer Aktivität, Arzneimitteln, die zur Behandlung einer Prostatahypertrophie verwendet werden, Steroidhormonen, Steroid-antientzündlichen Stoffen, nicht-selektiven und selektiven Betablockern, Calciumantagonisten, Benzodiazepinen.

3. Pflaster gemäß Anspruch 2, **dadurch gekennzeichnet, daß** es Oxybutynin als Wirkstoff enthält.

4. Pflaster gemäß Anspruch 2, **dadurch gekennzeichnet, daß** es einen Wirkstoff enthält, ausgewählt aus Terazosin und Finasterid.

5. Pflaster gemäß Anspruch 2, **dadurch gekennzeichnet, daß** es einen Wirkstoff enthält, ausgewählt aus Dehydroepiandrosteron und Östradiol.

6. Pflaster gemäß Anspruch 2, **dadurch gekennzeichnet, daß** es Norethisteron als Wirkstoff enthält.

7. Pflaster gemäß Anspruch 2, **dadurch gekennzeichnet, daß** es Ibuprofen als Wirkstoff enthält.

8. Pflaster gemäß Anspruch 2, **dadurch gekennzeichnet, daß** es Piroxicam als Wirkstoff enthält.

9. Pflaster gemäß Anspruch 2, **dadurch gekennzeichnet, daß** es Propanolol als Wirkstoff enthält.

10. Pflaster gemäß Anspruch 2, **dadurch gekennzeichnet, daß** es Atenolol als Wirkstoff enthält.

11. Pflaster gemäß Anspruch 2, **dadurch gekennzeichnet, daß** es Nifedipin als Wirkstoff enthält.

12. Pflaster gemäß Anspruch 2, **dadurch gekennzeichnet, daß** es Clonazepam als Wirkstoff enthält.

13. Pflaster gemäß Anspruch 2, **dadurch gekennzeichnet, daß** es Triazolam als Wirkstoff enthält.

14. Pflaster gemäß Anspruch 2, **dadurch gekennzeichnet, daß** es Lorazepam als Wirkstoff enthält.

15. Pflaster gemäß einem der Ansprüche 1 bis 14, **dadurch gekennzeichnet, daß** das Copolymer (a-3) ausgewählt ist aus der Gruppe bestehend aus:
i) Copolymeren vom kationischem Typ, basierend auf Dialkylaminoalkylmethacrylat und neutralen Alkylmethacrylatestern, wobei Alkyl einen C₁-C₁₀ linearen oder verzweigten Alkylrest bedeutet, wobei die Copolymere ein durchschnittliches Molekulargewicht zwischen 100.000 und 500.000 aufweisen und worin das Verhältnis von sich wiederholenden Dialkylaminoalkylmethacrylat/Neutralester-Einheiten zwischen 2:1 und 1:2 liegt;
ii) Copolymeren vom kationischen Typ, basierend auf Trialkylammoniumalkylmethacrylat und neutralen Alkylmethacrylatestern, neutralen Alkylacrylatestern, wobei Alkyl einen C₁-C₁₀ linearen oder verzweigten Alkylrest bedeutet, wobei die Copolymere ein durchschnittliches Molekulargewicht zwischen 1-00.000 und 500.000 aufweisen und worin das Alkylmethacrylat und Methylmethacrylat/Trialkylammoniumalkylmethacrylat-Verhältnis zwischen 40:1 und 20:1 liegt;
iii) Mischungen aus (i) und (ii).

16. Pflaster gemäß Anspruch 15, **dadurch gekennzeichnet, daß** das Copolymer ausgewählt wird aus der Gruppe bestehend aus:
a-3-1) Poly(butylmethacrylat, (2-Dimethylamino)methacrylat, Methylmethacrylat), worin das Verhältnis der drei Monomere jeweils 1:2:1 ist und durch ein durchschnittliches Molekulargewicht von 150.000 **gekennzeichnet** ist;
a-3-2) Poly(ethylacrylat, Methylmethacrylat, Trimethylammoniumethylmethacrylatchlorid), **gekennzeichnet durch** ein durchschnittliches Molekulargewicht von 150.000 und worin das Verhältnis der Monomere 1:2:0,2 beträgt;
a-3-3) Poly(ethylacrylat, Methylmethacrylat, Trimethylammoniumethylmethacrylatchlorid), **gekennzeichnet durch** ein durchschnittliches Molekulargewicht von 150.000 und worin das Verhältnis der Monomere 1:2:0,1 beträgt; und
a-3-4) Mischungen von zwei oder allen der Copolymere a-3-1), a-3-2), a-3-3).

17. Pflaster gemäß Anspruch 16, **dadurch gekennzeichnet, daß**, wenn der Wirkstoff vom basischen Typ ist, das Pflaster die Komponente (a-3-3) enthält.

18. Pflaster gemäß Anspruch 16, **dadurch gekennzeichnet, daß**, wenn der Wirkstoff vom sauren Typ ist, das Pflaster die Komponente (a-3-2) enthält.

19. Verfahren zur Herstellung des Pflasters gemäß einem der Ansprüche 1 bis 18, umfassend die folgenden Schritte:
α) Zugabe der Lösung des kationischen Polymers von Acryl- und/oder Methacrylestern, enthaltend Aminogruppen oder versalzte Ammoniumgruppen (a3) in einem organischen Lösungsmittel zu der Lösung von Silicon PSA in demselben organischen Lösungsmittel, das für (a3) verwendet wird,
β) Zugabe des Wirkstoffs zu der im vorstehenden Schritt (α) erhaltenen Mischung und Erhalt der resultierenden Mischung für 3 Stunden unter Rühren,
γ) Verteilen der Mischung, die sich aus dem vorstehenden Schritt (β) ergibt, auf dem Träger (b), Trocknen und Aufbringen des Schutzblatts (c) mit konventionellen Maschinen.

20. Verfahren gemäß Anspruch 19, **gekennzeichnet durch** die Verwendung von Lösungen von Siliconpolymeren in Ethylacetat, in welchem Fall das organische Lösungsmittel von Schritt (α) Ethylacetat ist.

21. Verfahren gemäß Anspruch 20, **dadurch gekennzeichnet, daß** die Lösungen der Siliconpolymere in Ethylacetat das Polymer in Konzentrationen von 60 Gew.% zum Gesamtgewicht der Lösung enthalten.

## Revendications

1. Timbre adapté à l'administration transdermique ou locale d'au moins un principe actif comprenant :
a) une matrice à base de polymères de silicone auto-collants contenant :
a-1) ledit principe actif dans des concentrations comprises entre 1 et 10% en poids du poids total de ladite matrice sèche collante ;
a-2) lesdits polymères de silicone dans une quantité comprise entre 80 et 98% en poids du poids total de ladite matrice sèche collante ;
a-3) au moins un copolymère de type cationique d'esters acryliques et/ou méthacryliques contenant des groupes amino ou des groupes de sels d'ammonium dans une concentration comprise entre 1 et 10% en poids du poids total desdits polymères de silicone adhésifs;
b) une couche support sur laquelle est disposée ladite matrice collante (a) ;
c) une couche protectrice disposée sur ladite matrice collante.

2. Timbre selon la revendication 1, **caractérisé en ce que** le principe actif est choisi dans le groupe comprenant les médicaments ayant une activité antispastique urinaire, les médicaments utilisés pour traiter l'hypertrophie prostatique, les hormones stéroïdiennes, les anti-inflammatoires stéroïdiens, les beta-bloquants sélectifs et non-sélectifs, les inhibiteurs calciques, les benzodiazépines.

3. Timbre selon la revendication 2, **caractérisé en ce qu'**il contient de l'oxybutynine en tant que principe actif.

4. Timbre selon la revendication 2, **caractérisé en ce qu'**il contient un principe actif choisi parmi la terazosine et le finasteride.

5. Timbre selon la revendication 2, **caractérisé en ce qu'**il contient un principe actif choisi parmi la déhydro-épiandrostérone et l'oestradiol.

6. Timbre selon la revendication 2, **caractérisé en ce qu'**il contient de la norethisterone en tant que principe actif.

7. Timbre selon la revendication 2, **caractérisé en ce qu'**il contient de l'ibuprofène en tant que principe actif.

8. Timbre selon la revendication 2, **caractérisé en ce qu'**il contient du piroxicam en tant que principe actif.

9. Timbre selon la revendication 2, **caractérisé en ce qu'**il contient du propranolol en tant que principe actif.

10. Timbre selon la revendication 2, **caractérisé en ce qu'**il contient de l'aténolol en tant que principe actif.

11. Timbre selon la revendication 2, **caractérisé en ce qu'**il contient de la nifedipine en tant que principe actif.

12. Timbre selon la revendication 2, **caractérisé en ce qu'**il contient du clonazepam en tant que principe actif.

13. Timbre selon la revendication 2, **caractérisé en ce qu'**il contient du triazolam en tant que principe actif.

14. Timbre selon la revendication 2, **caractérisé en ce qu'**il contient du lorazepam en tant que principe actif.

15. Timbre selon l'une quelconque des revendications 1 à 14, **caractérisé en ce que** le copolymère (a-3) est choisi dans le groupe comprenant :
i) les copolymères de type cationique à base d'esters dialkylaminoalkylméthacrylate, et les esters neutres d'alkylméthacrylate, dans lesquels le terme alkyle signifie un résidu en C₁ à C₁₀ linéaire ou ramifié, lesdits copolymères ayant une masse moléculaire moyenne comprise entre 100000 et 500000 et dans lesquels le rapport entre les unités répétitives esters dialkylaminoalkylméthacrylate / esters neutres est compris entre 2 :1 et 1 :2 ;
ii) les copolymères de type cationique à base d'esters trialkylammoniumalkylméthacrylate et les esters neutres d'alkylméthacrylate, les esters neutres d'alkylacrylates, dans lesquels le terme alkyle signifie un résidu en C₁ à C₁₀ linéaire ou ramifié, lesdits copolymères ayant une masse moléculaire moyenne comprise entre 100000 et 500000 et dans lesquels le rapport alkylméthacrylate et méthylméthacrylate / trialkylammoniumalkylméthacrylate est compris entre 40 :1 et 20 :1 ;
iii) des mélanges de i) et ii).

16. Timbre selon la revendication 15, **caractérisé en ce que** le copolymère est choisi dans le groupe comprenant :
a-3-1) le poly-(butylméthacrylate, (2-diméthylamino)-méthacrylate, méthylméthacrylate) dans lequel le rapport entre les 3 monomères est respectivement de 1 :2 :1, et qui est **caractérisé par** une masse moléculaire moyenne de 150000 ;
a-3-2) le poly(éthylacrylate, méthylméthacrylate, chlorure de triméthylammoniuméthyl-méthacrylate) **caractérisé par** une masse moléculaire moyenne de 150000 et dans lequel le rapport entre lesdits monomères est de 1 :2 :0,2 ;
a-3-3) le poly(éthylacrylate, méthylméthacrylate, chlorure de triméthylammoniuméthylméthacrylate) **caractérisé par** une masse moléculaire moyenne de 150000 et dans lequel le rapport entre lesdits monomères est 1 :2 :0,1 ; et
a-3-4) des mélanges de deux ou plus des copolymères a-3-1), a-3-2), a-3-3).

17. Timbre selon la revendication 16, **caractérisé en ce que** le principe actif est de type basique, ledit timbre contenant le composé (a-3-3).

18. Timbre selon la revendication 16, **caractérisé en ce que** le principe actif est de type acide, ledit timbre contenant le composé (a-3-2).

19. Procédé de préparation du timbre selon l'une quelconque des revendications 1 à 18, comprenant les étapes suivantes :
α) addition de la solution de polymère cationique d'esters acryliques et/ou méthacryliques contenant des groupes amino ou des groupes de sels d'ammonium (a3) dans un solvant organique à une solution de polymères de silicone auto-collants dans le même solvant organique que celui utilisé pour (a3),
β) addition du principe actif au mélange obtenu à l'étape précédente (α), et maintien sous agitation du mélange résultant pendant 3 heures,
γ) étalement sur le support (b) du mélange provenant de l'étape précédente (β), séchage et application de la couche protectrice (c) avec des machines conventionnelles.

20. Procédé selon la revendication 19, **caractérisé par** l'utilisation de solutions de polymères de silicone dans l'acétate d'éthyle, le solvant organique de l'étape (α) étant l'acétate d'éthyle.

21. Procédé selon la revendication 20, **caractérisé en ce que** lesdites solutions de polymère de silicone dans l'acétate d'éthyle contiennent ledit polymère dans une concentration de 60% en poids par rapport au poids total de ladite solution.
